# FASCICULE DE BREVET EUROPEEN

(11) **EP 0 594 835 B1**
(45) Date de publication et mention de la délivrance du brevet: **11.09.1996**
(21) Numéro de dépôt: 93911833.7
(22) Date de dépôt: 28.04.1993
(51) Int. Cl.: A61M 39/02

(54) **SYSTEME DE PERFUSION A DISPOSITIF D'ACCES IMPLANTABLE MULTICHAMBRE**
MEHRKAMMERIGE IMPLANTIERBARE INJEKTIONSSTELLE
INFUSION SYSTEM HAVING A MULTI-CHAMBER IMPLANTABLE ACCESS DEVICE

(30) Priorité: 29.04.1992 FR 9205311
(43) Date de publication de la demande: 04.05.1994
(73) Titulaire: CHRONOTEC S.A.R.L., F-06600 Antibes (FR)
(72) Inventeur: HAUSER, Jean-Luc, F-06600 Antibes (FR)
(74) Mandataire: Bonneau, Gérard Cabinet Bonneau Conseil en Propriété Industrielle
(86) Numéro de dépôt international: FR9300415
(87) Numéro de publication internationale: WO9321988

(56) Documents cités:
- EP-A- 0 241 159
- WO-A-89/10149
- WO-A-89/10157
- US-A- 4 705 501

## Description

### Domaine technique

La présente invention concerne un système de perfusion ayant un dispositif d'accès multichambre destiné a être implanté dans une zone sous-cutanée accessible du corps d'un patient en vue de constituer un site d'accès pour la perfusion de substances médicamenteuses ou le prélèvement d'un liquide physiologique du patient.

### Etat de la technique

La technique récente d'implantation de sites d'accès consiste à disposer, au cours d'une intervention sous anesthésie locale, une chambre sous le tissu sous-cutané de façon que celle-ci soit accessible à travers la peau ; cette chambre est reliée à un cathéter qui délivre une substance directement dans la région du corps concernée. Ces sites d'accès peuvent demeurer dans le corps du patient pendant des durées prolongées et permettent de supprimer des injections répétées intraveineuses, intra-artérielles, intrarachidiennes, intra-ventriculaires cérébrales ou intrapéritonéales et de les remplacer par de simples injections sous-cutanées.

Les sites d'accès sont de préférence formés d'une chambre dont l'accès se fait par une membrane épaisse ou septum à travers lequel on enfonce l'aiguille à perfusion. Le septum est auto-cicatrisant c'est-à-dire que la matière dont il est fait, par exemple du silicone, se referme dès que l'aiguille à perfusion a été retirée de sorte que la chambre conserve son étanchéité.

La technique ci-dessus s'est améliorée par l'emploi de sites d'accès comportant deux septum. En effet, il est quelquefois nécessaire de pratiquer deux perfusions simultanément avec deux substances médicamenteuses non miscibles. Pour ce faire on eu l'idée d'implanter un site d'accès comportant deux chambres juxtaposées aboutissant à deux conduits ou lumen du cathéter, et comportant également deux septum juxtaposés. Ce type de site d'accès peut aussi être utilisé pour effectuer simultanément une perfusion à travers un septum et un prélèvement d'un liquide physiologique àtravers l'autre septum parce que ce prélèvement est utile pour un examen ou bien parce qu'il est nécessaire d'effectuer certaines mesures physiologiques en même temps qu'on effectue la perfusion. Que ce soit dans un but d'examen ou dans un but d'analyse, on profite du cathéter en place plutôt que d'effectuer les prélèvements par voie intraveineuse, intra-artérielle, intrarachidienne ou autre.

Mais l'inconvénient du site d'accès à double septum tel que ci-dessus est la dimension importante du dispositif du fait des deux chambres juxtaposées. L'implantation exige une surface relativement importante, donc une poche sous-cutanée plus importante, ce qui peut entraîner des complications telles que des risques d'infection plus grands, sans compter une cicatrice assez grande du fait qu'il a fallu découper l'épiderme sur une plus grande largeur.

C'est pourquoi il existe maintenant des sites d'accès à double septum comportant deux chambres superposées tels que décrits dans les brevets US-A-4.705.501, WO-A-89/10149 et WO-A-89/10157. Mais ces dispositifs présentent l'inconvénient d'utiliser soit deux aiguilles séparées, soit une seule aiguilles comportant deux lumen.

En effet, l'inconvénient majeur des deux aiguilles séparées, en plus du danger de déplacement d'une des aiguilles entraînant son retrait pendant la perfusion (ou extravasation), avec les dangers de fuite sous-cutanée qui peuvent en résulter, provient de la possibilité que l'aiguille qui doit atteindre le septum inférieur, ne soit pas suffisamment enfoncée. Dans ce cas, il y aura mélange des deux médicaments à perfuser et risque de réaction ou d'incompatibilité entre les deux médicaments entraînant un danger pour le patient, ou, s'il s'agit d'un prélèvement effectué simultanément avec une perfusion, mélange du médicament à perfuser et du liquide physiologique à prélever.

L'utilisation d'une seule aiguille comportant deux lumen réunit les deux inconvénients mentionnés ci-dessus, à savoir le risque d'extravasation dû à l'arrachement inopportun de l'aiguille combiné au mélange des liquides des chambres provoqué par cet arrachement pendant la perfusion.

### Expose de l'invention

L'invention a donc pour but de remédier aux inconvénients ci-dessus grâce à un dispositif d'accès implantable multichambre ne posant pas plus de problèmes pour son implantation qu'un dispositif d'accès à une seule chambre.

Un autre but de l'invention est de fournir un dispositif d'accès implantable à plusieurs chambres superposées combiné à un dispositif de perfusion à plusieurs aiguilles solidaires empêchant tout risque d'arrachement pouvant entraîner une extravasation.

L'objet de l'invention est donc un système de perfusion dont le dispositif de perfusion comporte une pluralité d'aiguilles correspondant à la pluralité de chambres du dispositif d'accès, les aiguilles étant solidaires, parallèles, proches les unes des autres et de longueur différente de manière à ce que l'accès à la chambre la plus éloignée dans le sens de pénétration des aiguilles par l'aiguille la plus longue entraîne l'accès aux autres chambres par respectivement chacune, des autres aiguilles.

### Brève description

Les buts, objets et caractéristiques de l'invention seront mieux compris à la lecture de la description qui suit faite en référence aux dessins dans lesquels :
la figure 1 représente une vue en coupe d'un dispositif d'accès à deux chambres selon l'invention,
la figure 2 représente un dispositif à deux aiguilles de perfusion ou de prélèvement utilisé conjointement avec le dispositif d'accès illustré sur la figure 1.

### Description détaillée

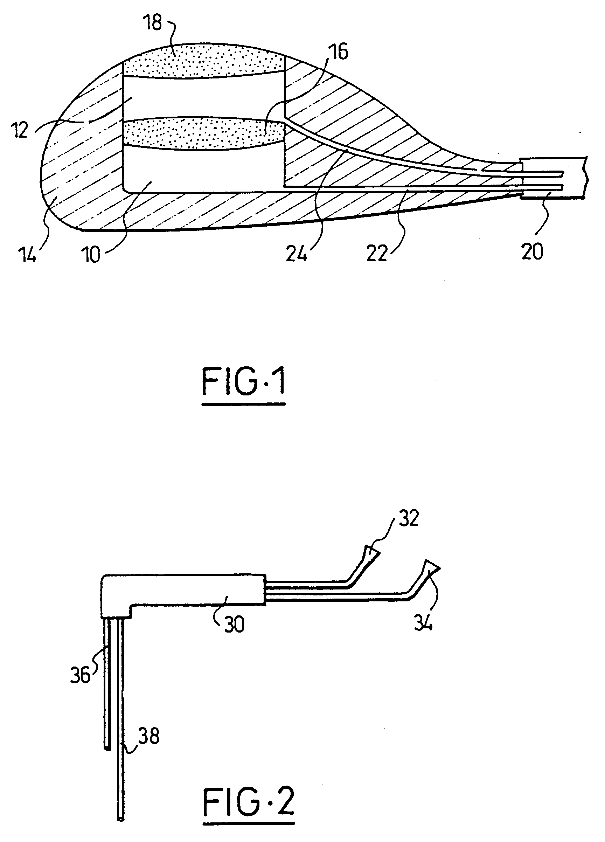

Le dispositif représenté sur la figure 1 est un dispositif d'accès à deux chambres selon un mode de réalisation préféré de l'invention. Ce dispositif ou site d'accès, implanté sous la peau du patient par une intervention généralement sous anesthésie locale, comprend deux chambres superposées 10 et 12 disposées dans une structure 14, de préférence en matière plastique. La chambre inférieure 10 dans le sens de pénétration des aiguilles est fermée par une membrane épaisse ou septum 16, et la chambre supérieure 12 dans le sens de pénétration des aiguilles est fermée par une membrane épaisse ou septum 18. Le site d'accès est relié à un cathéter 20 se trouvant également dans le corps du patient, par des conduits 22 et 24 ouvrant respectivement dans la chambre inférieure 10 et la chambre supérieure 12. Les deux conduits 22 et 24 sont prolongés dans le cathéter 20 par deux lumen non communicants.

Chaque septum 16 ou 18 est auto-cicatrisant, c'est-à-dire qu'après avoir été pénétré par une aiguille de perfusion ou prélèvement, il redevient étanche après le retrait de l'aiguille. Cette propriété du septum bien connue de l'homme du métier, permet de maintenir chaque chambre étanche, et empêcher ainsi le mélange des substances médicamenteuses se trouvant dans les chambres après la perfusion ou lors du prélèvement.

Bien que la figure 1 représente un mode de réalisation préféré de l'invention, il est clair que différentes modifications peuvent lui être apportées sans sortir du cadre de l'invention. Ainsi, même si l'existence de deux chambres constitue le mode de réalisation préféré, on peut prévoir trois chambres ou plus superposées ou deux groupes juxtaposés de deux (ou plus) chambres superposées ou encore deux chambres superposées, juxtaposées à une chambre unique. Ces réalisations, à la portée de l'homme du métier, n'ont pas été représentées sur les dessins. De même, bien que les chambres représentées sur la figure 1 aient une profondeur constante, on peut très bien concevoir des chambres dont la profondeur soit variable, ce qui rend le site d'accès incliné (de gauche à droite sur la figure 1) vers le cathéter.

Sur la figure 2 est représenté le dispositif multiaiguilles utilisable notamment avec le dispositif d'accès illustré sur la figure 1. Ce dispositif comporte une armature rigide 30, de préférence en métal, contenant deux conduits dont l'un est en communication grâce à l'embouchure d'accès 32 avec une ligne de perfusion ou de prélèvement, et l'autre est en communication grâce à l'embouchure d'accès 34 avec une autre ligne de perfusion ou de prélèvement. L'armature rigide 30 est recourbée à 90 % à son autre extrémité qui se prolonge par deux aiguilles 36 et 38 respectivement en communication avec les embouchures d'accès 32 et 34.

Les deux aiguilles 36 et 38 forment avec l'armature 30 un ensemble solidaire. Elles sont de préférence-parallèles, espacées légèrement d'environ 3 à 5 mm, et de longueur différente. Elles pourraient également être plus espacées pour être adaptées à l'introduction dans des chambres juxtaposées. L'aiguille 36 est plus courte que l'aiguille 38 de sorte que, lorsque les aiguilles sont enfoncées dans le site d'accès représenté sur la figure 1, l'aiguille la plus longue 38 traverse le septum 16 et son extrémité atteint la chambre 10 pendant que l'aiguille la plus courte 36 traverse le septum 18 et que son extrémité atteint la chambre 12. Ainsi, deux perfusions peuvent être pratiquées simultanément, par exemple pour l'injection simultanée de deux substances non miscibles. Mais le dispositif de la figure 2 peut également être utilisé pour pratiquer deux prélèvements simultanés, et surtout pour pratiquer un prélèvement de liquide physiologique pour analyse ou examen au moyen de l'une des deux chambres, pendant qu'une perfusion est pratiquée au moyen de l'autre chambre.

L'avantage essentiel du système de perfusion formé par le dispositif d'accès multichambre et le dispositif multiaiguilles selon la présente invention, est obtenu grâce à la présence des deux (ou plusieurs) aiguilles solidaires combiné au fait que l'aiguille la plus longue traverse les deux septum pour avoir accès à la chambre inférieure. En effet, le septum formé généralement d'une matière plastique type silicone exerce une certaine pression autour de l'aiguille. Mais avec les sites d'accès de la technique antérieure utilisant soit deux aiguilles séparées, soit une seule aiguille à deux lumen, le déplacement latéral de l'aiguille avec le risque d'extravasation qui en résulte est toujours possible. Par contre, le dispositif de perfusion à deux aiguilles représenté sur la figure 2, est maintenu en place grâce aux forces de friction qui s'exercent sur chacune des aiguilles (l'aiguille la plus longue est maintenue non seulement à son extrémité mais également en son milieu à la traversée du septum de la chambre supérieure), ce qui évite tout déplacement possible des deux aiguilles. Ce maintien en place dû aux plus grandes forces de friction est renforcé par l'impossibilité pour les aiguilles d'effectuer des rotations sur elles-mêmes qui sont souvent la cause d'une extravasation dans les dispositifs de la technique antérieure.

Bien que l'invention ait été décrite en référence à un mode de réalisation dans lequel le dispositif est implanté dans le corps du patient, il est évident que les principes de l'invention sont également applicables à un cathéter externalisé.

## Revendications

1. Système de perfusion comprenant un dispositif d'accès multichambre implantable dans le corps humain et un dispositif de perfusion à aiguilles destiné à pratiquer une perfusion ou tout autre intervention appropriée, ledit dispositif d'accès multichambre comportant une pluralité de chambres indépendantes (10, 12) superposées dans la direction de pénétration des aiguilles, et l'accès à chacune desdites chambres se faisant par un septum (16, 18);
ledit système étant caractérisé en ce que ledit dispositif de perfusion comporte une pluralité d'aiguilles (36, 38) correspondant à ladite pluralité de chambres du dispositif d'accès, lesdites aiguilles étant solidaires, parallèles, proches les unes des autres et de longueur différente de manière à ce que l'accès à la chambre la plus éloignée dans le sens de pénétration des aiguilles par l'aiguille la plus longue (38) entraîne l'accès aux autres chambres par respectivement chacune des autres aiguilles, de telle sorte que les forces de friction exercées sur chacune des aiguilles contribuent à empêcher tout mouvement latéral dudit dispositif de perfusion pouvant entraîner un risque d'extravasation.

2. Système de perfusion selon la revendication 1, dans lequel ledit dispositif d'accès multichambre comporte deux premières chambres (10, 12) superposées dans la direction de pénétration des aiguilles.

3. Système de perfusion selon la revendication 2, dans lequel ledit dispositif d'accès multichambre comoporte en outre deux autres chambres superposées dans la direction de pénétration des aiguilles, lesdites autres chambres étant juxtaposées auxdites deux premières chambres (10, 12).

4. Système de perfusion selon la revendication 2, dans lequel ledit dispositif d'accès multichambre comporte en outre une chambre juxtaposée auxdites deux premières chambres (10, 12).

## Claims

1. Infusion system comprising a multi-chamber access device implantable in the human body and an infusion device with needles for carrying out an infusion or any other appropriate intervention, said multichamber access device including a plurality of independent chambers (10, 12) arranged one above the other in the direction of the needle penetration, and the access to each of said chambers being via a septum (16, 18) ;
said system being characterized in that said infusion device comprises a plurality of needles (36, 38) corresponding to said plurality of chambers of the access device, near to one another and of different lengths so that the access to the most remote chamber in the direction of needle penetration by the longest needle (38) causes the access to the other chambers by respectively each of the other needles, whereby the friction forces exerted on each of the needles help preventing said infusion device from any lateral movement capable to raise a risk of estravasation.

2. Access device according to claim 1, wherein said multi-chamber access device includes two first chambers (10, 12) arranged one on top of the other in the direction of the needle penetration.

3. Infusion system according to claim 2, wherein said multi-chamber access device further comprises two other chambers arranged one on top of the other in the direction of the needle penetration and placed side by side to said two first chambers (10, 12).

4. Infusion system according to claim 2, wherein said multi-chamber access device further comprises a chamber placed side by side to said two first chambers (10, 12).

## Patentansprüche

1. Infusionssystem, umfassend eine in den menschlichen Körper implantierbare Mehrkammern-Eintrittsvorrichtung und eine Nadel-Infusionsvorrichtung, die dazu bestimmt ist, eine Infusion oder jede andere geeignete Intervention vorzunehmen, wobei diese Mehrkammern-Eintrittsvorrichtung eine Vielzahl von unabhängigen Kammern (10, 12) aufweist, die in der Eindringrichtung der Nadeln übereinander angeordnet sind, und der Eintritt in jede dieser Kammern über ein Septum (16, 18) vor sich geht,
wobei dieses System dadurch gekennzeichnet ist, daß diese Infusionsvorrichtung eine dieser Vielzahl von Kammern der Eintrittsvorrichtung entsprechende Vielzahl von Nadeln (36, 38) besitzt, die miteinander fest verbunden, zueinander parallel, einander nahe und von unterschiedlicher Länge sind, so daß der Eintritt der längsten Nadel (38) in die in der Eindringrichtung der Nadeln entfernteste Kammer den Eintritt jeweils jeder der anderen Nadeln in die anderen Kammern mit sich bringt, so daß die auf jede der Nadeln ausgeübten Reibungskräfte dazu beitragen, jede seitliche Bewegung dieser Infusionsvorrichtung zu verhindern, die eine Gefahr der Extravasation mit sich bringen kann.

2. Infusionssystem nach Anspruch 1, in dem diese Mehrkammern-Eintrittsvorrichtung zwei erste Kammern (10, 12) aufweist, die in der Eindringrichtung der Nadeln übereinander angeordnet sind.

3. Infusionssystem nach Anspruch 2, in dem die Mehrkammern-Eintrittsvorrichtung außerdem zwei weitere in der Eindringrichtung der Nadeln übereinander angeordnete Kammern aufweist, die neben diesen beiden ersten Kammern (10, 12) angeordnet sind.

4. Infusionssystem nach Anspruch 2, in dem diese Mehrkammern-Eintrittsvorrichtung außerdem eine Kammer besitzt, die neben diesen beiden ersten Kammern (10, 12) angeordnet ist.
